# EUROPEAN PATENT SPECIFICATION

(11) **EP 1 027 067 B1**
(45) Date of publication and mention of the grant of the patent: **01.09.2004**
(21) Application number: 98912861.6
(22) Date of filing: 26.03.1998
(51) Int. Cl.: A61K 38/11

(54) **USE OF SUBSTANCES HAVING OXYTOCIN ACTIVITY FOR THE PREPARATION OF MEDICAMENTS FOR THE TREATMENT OF AFFECTIVE PAIN SYNDROMES**
VERWENDUNG VON SUBSTANZEN MIT OXYTOCINWIRKUNG ZUR HERSTELLUNG EINES ARZNEIMITTELS ZUR BEHANDLUNG AFFEKTIVER SCHMERZSYNDROME
UTILISATION DE SUBSTANCES A ACTIVITE OXYTOCIQUE POUR LA PREPARATION DE MEDICAMENTS DESTINES AU TRAITEMENT DE SYNDROMES DOULOUREUX PSYCHOSOMATIQUES

(30) Priority: 27.03.1997 SE 9701161
(43) Date of publication of application: 16.08.2000
(73) Proprietor: EntreTech Medical AB, 200 74 Malmö (SE)
(72) Inventor: ALFVOEN, Gösta, S-11620 Stockholm (SE); LUNDEBERG, Thomas, S-18131 Lidingö (SE); UVNAES-MOBERG, Kerstin, S-18262 Djursholm (SE)
(74) Representative: Berg, Sven Anders
(86) International application number: PCT/SE1998/000553
(87) International publication number: WO 1998/043660

(56) References cited:
- GUT, Volume 39, 1996, D. LOUVEL et al., "Oxytocin Increases Thresholds of Colonic Visceral Perception in Patients with Irritagle Bowel Syndrome", pages 741-747.
- ACTA PHYSIOL. SCAND., Volume 144, 1992, K. UVNAS-MOBERG et al., "Oxytocin Increases and a Specific Oxytocin Antagonist Decreases Pain Threshold in Male Rats", pages 487-488.
- DONALD D. PRICE: "Psychological and Neural Machanisms of the Affective Dimension of Pain", SCIENCE, , -09. June 2000, vol. 288, no. , pages 1769 to 1772

### SEQUENCE LISTING

<110> ENTRETECH MEDICAL AB
<120> USE OF SUBSTANCES HAVING OXYTOCIN ACTIVITY FOR PREPARATION OF MEDICAMENTS FOR THE TREATMENT OF AFFECTIVE PAIN SYNDROMES
<130> 53688
<140>
<141>
<160> 8
< 170> PatentIn Ver. 2.1
<210> 1
<211> 9
<212> PRT
<213> Artificial Sequence
<220>
<221> MOD_RES
<222> (9)
<223> AMIDATION
<220>
<221> DISULFID
<222> (1)..(6)
<220>
<223> Xaa(3)=Cha
<220>
<223> Xaa(4)=Cit
<220>
<223> Description of Artificial Sequence:PEPTIDE
<400> 1
   Cys Tyr Xaa Xaa Asn Cys Pro Arg Gly
   1 5
<210> 2
<211> 9
<212> PRT
<213> Artificial Sequence
<220>
<221> MOD_RES
<222> (9)
<223> AMIDATION
<220>
<221> DISULFID
<222> (1)..(6)
<220>
<223> Description of Artificial Sequence:PEPTIDE
<400> 2
   Cys Tyr Val Thr Asn Cys Pro Leu Gly
   1 5
<210> 3
<211> 9
<212> PRT
<213> Artificial Sequence
<220>
<221> MOD_RES
<222> (9)
<223> AMIDATION
<220>
<221> DISULFID
<222> (1)..(6)
<220>
<223> Xaa(3)=Hoph
<220>
<223> Description of Artificial Sequence: PEPTIDE
<400> 3
   Cys Tyr Xaa Thr Asn Cys Pro Val Gly
   1 5
<210> 4
<211> 9
<212> PRT
<213> Artificial Sequence
<220>
<221> MOD_RES
<222> (9)
<223> AMIDATION
<220>
<221> DISULFID
<222> (1)..(6)
<220>
<223> Xaa(4)=Cit
<220>
<223> Description of Artificial Sequence:PEPTIDE
<400> 4
   Cys Tyr Phe Xaa Asn Cys Pro Leu Gly
   1 5
<210> 5
<211> 9
<212> PRT
<213> Artificial Sequence
<220>
<221> MOD_RES
<222> (9)
<223> AMIDATION
<220>
<221> DISULFID
<222> (1)..(6)
<220>
<223> Xaa(3)=Cha
<220>
<223> Xaa(8)=Hos
<220>
<223> Description of Artificial Sequence:PEPTIDE
<400> 5
   Cys Tyr Xaa Arg Asn Cys Pro Xaa Ala
   1 5
<210> 6
<211> 9
<212> PRT
<213> Artificial Sequence
<220>
<221> MOD_RES
<222> (9)
<223> AMIDATION
<220>
<221> DISULFID
<222> (1)..(6)
<220>
<223> Xaa(4)=Daba
<220>
<223> Xaa(8)=Daba
<220>
<223> Description of Artificial Sequence:PEPTIDE
<400> 6
   Cys Tyr Val Xaa Asn Cys Pro Xaa Ala
   1               5
<210> 7
<211> 9
<212> PRT
<213> Artificial Sequence
<220>
<221> MOD_RES
<222> (9)
<223> AMIDATION
<220>
<221> DISULFID
<222> (1)..(6)
<220>
<223> Xaa(3)=Hoph
<220>
<223> Xaa(4)=Daba
<220>
<223> Xaa(8)=Cit
<220>
<223> Description of Artificial Sequence:PEPTIDE
<400> 7
   Cys Tyr Xaa Xaa Asn Cys Pro Xaa Ala
   1 5
<210> 8
<211> 9
<212> PRT
<213> Artificial Sequence
<220>
<221> MOD_RES
<222> (9)
<223> AMIDATION
<220>
<221> DISULFID
<222> (1)..(6)
<220>
<223> Description of Artificial Sequence:PEPTIDE
<400> 8
   Cys Tyr Phe Arg Asn Cys Pro Val Ala
   1 5

## Description

The present invention relates to a new use of oxytocin.

### Background of the invention

Pain syndromes which have no readily identifiable cause are characterized by chronic pain for which no abnormality can be demonstrated either in histological sections or in the physiology of the affected tissues. Fibromyalgia syndrome and myofascial pain syndrome are two such complex conditions for which there is still no useful treatment. Other pain syndromes of non-organic origin are recurrent abdominal pain in children and different types of colic or irritable bowel syndrome.

Fibromyalgia syndrome is a common clinical pain disorder in which palpable fibrositic points, FTP, are associated with generalised muscular aching, morning stiffness, fatigue and nonrestorative sleep. Widespread pain which can be described as chronic muscular aching in all parts of the body is the dominating feature of the disease and the presence of FTP distinguishes from other diseases in the category soft tissue rheumatism. Many of the patients also present more or less serious irritable bowel syndromes. Fibromyalgia mainly affects women between 40 and 45 years of age.

The pathophysiology of fibromyalgia is unknown. Theories about its aetiology fall into four main categories, that is sleep disturbances, muscle abnormalities, neuropeptide changes and immune system alterations. It is commonly believed that the microcirculation is impaired in patients with fibromyalgia. It has been suggested that the chronic pain and point tenderness can be ascribed to a disorder of the pain-sensing pathways in the nervous system. Serotonin, that is 5-hydroxytryptamine, and noradrenaline are central nervous system neurotransmitters which might be involved in the generation or maintaining of fibromyalgia.

Drug therapy is often insufficient for patients with fibromyalgia. Analgesics and antiinflammatory agents do not give any relief There are, however, completed clinical trials showing that some antidepressants, such as amitriptyline and cyclo-benzaprine, can be effective in at least a minor group of patients. A disadvantage with even low doses of these drugs is that they are often poorly tolerated; common side-effects are drowsiness, agitation and gastrointestinal upset. There are also other antidepressants, that is serotonin receptor antagonists which have no or little effect on the symptoms and signs of fibromyalgia. Alprazolam, a triazalobenzodiazepine, approved for the treatment of anxiety and depression, gave together with ibuprofen a significant greater than 30% improvement in half of the patients. The drop out rate was greater than expected and the improvements were slow to occur, for some patients 16 weeks of treatment were required. Cognitive behavioural therapy in conjunction with aerobic exercise, physiotherapy, biofeedback training and relaxation therapy may be of benefit at least to selected patients, but will not constitute a solution. (Wall, P.D., Melzack, R., eds, Pain, Churchill Livingstone, 1995, McCain, G.A., Fibromyalgia and myofascial pain syndromes.)

Navel colic or recurrent abdominal pain of non-organic origin, RAP, is a common, probably psychosomatic disorder of childhood, which causes much distress due to pain, worry and social inhibition. The diagnostic criteria are vague and based more on exclusion than on positive clinical and laboratory signs. The classical definition of RAP, as given by Apley J. (The child with abdominal pains, Oxford, Blackwell, 1975), is as follows: "A period of three or more episodes of abdominal pain occurring over a period of at least 3 months, affecting the child's activities, yet where no sign of organic disease is found".

Today the general opinion is that RAP has a psychosomatic origin, that is bodily symptoms of psychic, emotional or mental origin. There are however no clearcut answers. Life events, stress and depression play a part but seem to explain only some of the causes of RAP.

The most prominent feature of this disorder is, as its name implies, recurrent abdominal pains, which may vary in intensity between mild and intense pain, in duration between a few seconds and whole days, and in frequency between less than one attack per month and several attacks per day. The pain focuses mainly around the umbilicus. Also other pains, such as headache, limb pains and chest pains are common among RAP children. Gastrointestinal symptoms are also very common, such as loss of appetite, nausea, vomiting, obstipation and mild diarrhea. It has been demonstrated that children with recurrent abdominal pain have tense and tender abdominal wall muscles, especially around the navel, owing to an increased muscular tension (Acta Paediatr. 1993; 82: 400-3, Alfvén G., Preliminary findings on increased muscle tension and tenderness, and recurrent abdominal pain in children. A clinical study). Traits such as introversion and susceptibility to anxiety may be common among RAP children. The complex clinical picture including endocrinological deviances, as well as muscular symptoms points to a psychosomatic origin.

There are different types of colic, some of which do not respond to analgesics or opioids. Said colic conditions can be described as a cramp and pain condition in the colon which e.g. are caused by the formation of gas and accompanying expansion of the intestines, resulting in muscular cramp and dysfunction. Infant colic is one such condition. Irritable bowel syndrome, IBS, or RAP in adulthood is a related condition referring to a complex of symptoms from the gastrointestinal tract, such as bowel pain, disordered defecation habits and flatus.

There are several different types of pain. In case of a tissue damage or lesion, pain receptors, so called nociceptors, are activated by mechanical, thermal or chemical stimuli. The information is transported through nerves, the spinal cord and the brain stem to the brain cortex where the pain is experienced. Pain can also arise anywhere in the pain pathway or by damage to the control systems affecting the transmission of the pain signals in the cord or in the ascending systems, that is the brain stem and the thalamus. From the thalamus impulses are led to the sensoric area of cortex, as well as to the limbic system, the part of the brain that controls emotional reactions. Pain is generally divided into four different types, that is nociceptive, neurogenic, idiopathic and psychogenic (Amér, S., Differentiation of pain and treatment efficacy, Thesis Karolinska Institutet, Stockholm, Sweden, 1991). A nociceptive pain is caused by tissue damage, a neurogenic pain by a damage or malfunction of the nervous system, a psychogenic pain by a psychic disorder or disturbance, and the origin of idiopathic pain is still unknown.

It is well known that pain except its classification into nociceptive, neurogenic, idiopathic and psychogenic pain also may be classified by its two main components: sensory and affective (Price, DD, et al., Pain 17:45-56, 1983). The sensory component deals with the intensity of the perceived pain and is projected on the gyrus cinguli, thalamus and the somatosensory cortex. The affective component on the other hand refers to the perception of pain and is projected in amygdala, hippocampus, hypothalamus, locus ceruleus (LC) and nucleus tractus solitarius (NTS) and accompanied by activity in the frontal cortex, the so called associative cortex. The sensory component of pain may be studied by reflex methodology as used in the study by Kurosawa et al., Regulatory Peptides, 1998 (in press). It is important to stress that drugs affecting the sensory component, e.g. local anaesthetics, NSAIDs etc may leave the affective component unaltered/unchanged and vice versa. In an experimental study on the effects of oxytocin on the sensory aspects of IBS pain it is demonstrated that it prolongs the withdrawal latencies, a finding interpreted as modulation of nociception. This finding of changes in reflex responses is of theoretical interest, but has no relevance to the finding of reduced pain (affective component) reported in our patent. Experimental studies using PET have demonstrated that the pain associated with IBS is projected to the frontal cortex, i.e. being mainly of an affective nature. To elucidate if oxytocin has any influence on the central sensory components of pain, studies using somatosensory evoked potentials (SEP) were carried out in rats. Nociceptive stimulation was applied to the skin, muscle and viscera and the evoked potentials recorded. Oxytocin was then administered either subcutaneously, intravenously or intrathecally in doses previously shown to relieve nociceptive behaviour. The administration of oxytocin did not effect the SEPs, i.e. oxytocin has no effect on the central projections or the sensory component of pain. Taken together our results are the first to demonstrate that oxytocin affects the affective component of pain, the component being of relevance in fibromyalgia and IBS (Ward et al., Pain 7:331-349, 1979). Furthermore, it has been demonstrated that the affective component of pain is related to anxiety and depression. This is of particular interest as we have found that oxytocin as well as sedatives and antidepressants induces anxiolysis utilizing similar mechanisms. The two latter also being used for the alleviation of fibromyalgia and IBS.

GUT, Volume 39, 1996, D. LOUVEL et al. "Oxytocin Increases Thresholds of Colonic Visceral Perception in Patients with Irritable Bowel syndrome", pages 741-747, discloses that oxytocin may have an antinociceptive effect which means that the sensory (intensity) component of pain is alleviated by oxytocin administration which is demonstrated by using animal experiments. The invention, however, does not relate to the sensory component but only to the affective (unpleasantness) component of pain. This pain component cannot be measured using animal experiments. Furthermore, it has been shown that the sensory and affective pain components have very different biological background and project to different areas of the brain (Price, Review: Neuroscience Vol. 288, p. 1769-1772 (2000)).

As it previously has been reported that the expression of oxytocin receptors is influenced (increased) by estrogen, female rats were treated with a combination of estrogen and oxytocin and the anxiolytic effect was investigated. The results show that this combination potentiated the anxiolytic effects. In two female patients with fibromyalgia obtaining minor relief with i.n. administration of oxytocin, estrogen supplementation was added. Both patients reported a more marked alleviation of their pain.

It has recently been shown that vagal and sympathetic afferent nerves also may contribute to the sensation of pain and illness behaviour such as anxiety, muscle soreness, sleep disturbances etc. This could be an indication that the vagal and/or sympathetic pathway may contribute to pain in the above mentioned pain syndromes. This would also explain why previously used treatments, such as with opioids, NSAIDs etc are ineffective.

There are several mechanisms in the human body for the reduction of pain, for instance the ability to produce endorphins. Certain transmitter substances of importance in psychic depression, especially serotonin, also have an influence on the sensitivity of the pain pathway. Treatment with analgesics is effective to reduce pain caused by injury, for instance a fracture or inflammation. Said substances, however, have no effect on neurogenic or psychogenic pain for which antidepressive drugs sometimes might have a pain reducing effect, nor an effect on idiopathic pain. It is also well known that patients suffering from idiopathic pain do not respond to opioids.

Today there is no treatment available resulting in a complete relief of idiopathic pain in the conditions described above.

### Prior art

Samborski et al., Biochemische Veränderungen bei der Fibromyalgie, Z Rheumatol, 1996: 55(3), 168-73 reports that patients with fibromyalgia revealed a significantly decreased level of e.g. serotonin and a significantly increased level of prolactin in blood. For oxytocin no significant difference could be observed.

Children with recurrent abdominal pain of non-organic origin have been shown to have reduced concentrations of oxytocin and cortisol in plasma, but no significant difference in prolactin level compared to a group of control children; Alfvén G., de la Torre B., Uvnäs Moberg K. Depressed plasma concentrations of cortisol and oxytocin in children with recurrent abdominal pain of non-organic origin Acta Paediatr., 83(10), 1076-80, 1994.

An anti-nociceptive effect of oxytocin has been demonstrated in rats after s.c. administration, which effect can be reversed by the opioid antagonist naloxone; Petersson et al., Oxytocin increases nociceptive thresholds in a long-term perspective in female and male rats, Neuroscience Letters 212, 87-90, 1996. It is suggested that oxytocin increases the synthesis and release of endogenous opioids and might be used as an analgesic. Pain-relieving effects have also been shown in humans in patients with low back pain and in patients with cancer pain. The type of pain concerned in these cases obviously is a nociceptive and/or neurogenic pain and there is nothing indicating that oxytocin could have an effect also on idiopathic pain. It is important to stress that pain has many different causes which explains why different treatments may have a pain relieving effect. Nociceptive pains like low back pain and cancer pain respond to opioids. Low back pain and cancer pain of neurogenic origin have been shown to respond to opioids but to a lesser degree.

Oxytocin was one of the first peptide hormones to be isolated and sequenced. It is a nonapeptied with two cysteine residues that form a disulfide bridge between positions 1 and 6 and corresponds to the formula

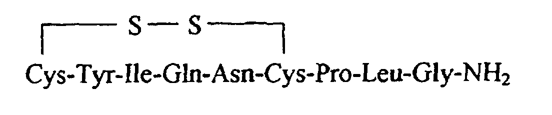

For a long time the only effects attributed to oxytocin were its stimulating effects on milk ejection and uterine contractions, but in the past decades it has been shown that oxytocin exerts a wide spectrum of effects within the CNS. It has been suggested that oxytocin participates in the control of memory and learning processes and of various types of behaviour such as feeding, locomotion, as well as maternal and sexual behaviour. Oxytocin is also suggested to participate in the control of cardiovascular functions, thermoregulation, pain threshold and fluid balance. There is also evidence that oxytocin is involved in the control of various immunological processes. It has recently been demonstrated that oxytocin injections cause a lowering of blood pressure and increased weight gain long-lasting effects after repetitive administration.

### Description of the invention

It has now surprisingly been found that substances with oxytocin activity have a pain relieving effect in patients suffering from an affective pain syndrome. As it is well known that affective pain is not alleviated by opioids the obtained effect was highly unexpected and implicates that said pain relief is mediated by other factors.

Idiopathic pain, such as in fibromyalgia, has for a long time been considered to be one of the most therapeutic resistant forms of pain. The effects obtained with oxytocin are surprising also because all other forms of interventions tried have proven to be either ineffective or to have only a limited effect in a limited number of patients.

The invention refers to the use of substances with oxytocin activitiy for the preparation of a pharmaceutical composition for treatment or prophylaxis of affective pain syndromes of non-organic origin in fibromyalgia, myofascial pain syndrome, navel colic, colitis and affective components associated with pain of organic origin in mammals including man.

A preferred use of the invention is for the treatment of fibromyalgia.

Another preferred use is for the treatment of navel colic.

There are different processes described for the synthetical production of oxytocin; commercial processes are for instance described in US patents 2,938,891 and 3,076,797.

In the human body oxytocin is produced in the para-ventricular nucleus, PVN, and the supraoptic nucleus, SON, of the hypothalamus. It differs by only two amino acids from vasopressin, which is also produced in these nuclei. The magnocellular oxytocinergic neurons of the SON and PVN send projections into the posterior pituitary gland from which oxytocin or vasopressin are secreted into the circulation. Parvocellular neurons that originate in the PVN project into multiple areas within the central nervous system, CNS. The oxytocin-producing cells are innervated by cholinergic, catecholaminergic as well as peptidergic neurons. The presence of oxytocin in different tissues outside the brain, such as the uterus, ovaries, testis, thymus, adrenal medulla and pancreas has been demonstrated and oxytocin is suggested to exert local effects in these organs.

A parallel secretion of oxytocin into the brain regions and into the circulation occurs in response to some stimuli such as suckling, but other stimuli may cause separate activation of oxytocinergic neurons, terminating in the brain or the pituitary.

In this context oxytocin refers, whenever applicable, in addition to oxytocin also to precursors, metabolic derivatives, oxytocin agonists or analogues displaying the same properties.

Substances with oxytocin activity according to the invention can be described by the general formula I 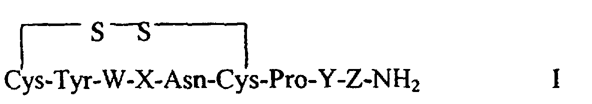 wherein
W is selected from the group consisting of Ile, Cha, Val, Hoph and Phe;
X is selected from the group consisting of Gln, Ser, Thr, Cit, Daba and Arg;
Y is selected from the group consisting of Leu, Ile, Arg, Hos, Daba, Cit and Val; where Leu, Ile and Val give oxytocin analogues and Arg, Hos, Daba and Cit give vasopressin analogues; and
Z is selected from the group consisting of Gly and Ala.

The unnatural amino acids in said substances have the following structures:

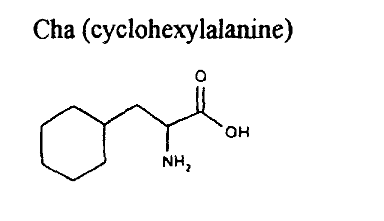

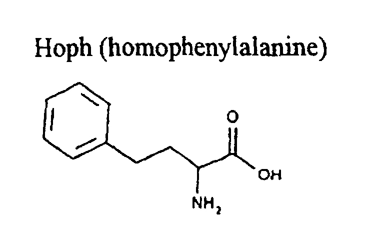

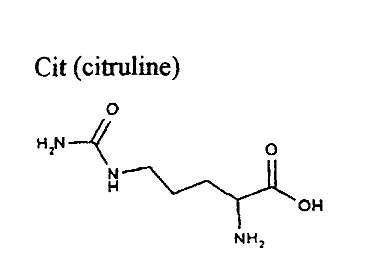

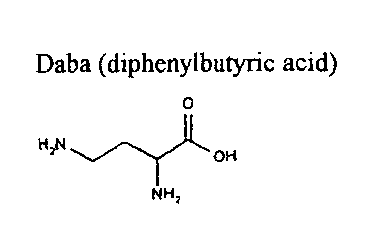

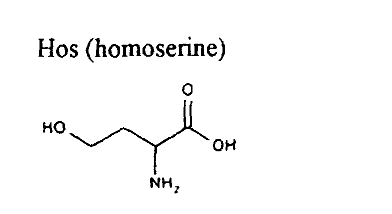

Said amino acids are all commercially available, for instance from Bachem and Sigma.

The amino acids in the substances according to the invention can be either L-or D-amino acids.

Substances according to the invention also include nonapeptides having sequences with reversed peptide bonds. These sequences are preferably inverted sequences; more preferably comprising D-amino acids.

The nonapeptides of the formula I are believed to present oxytocin activity owing to the structural similarity to oxytocin having the formula:

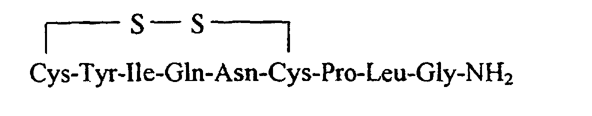

In the oxytocin structure the positions 1, 2, 5, 6 and 7 have remained unchanged, that is the disulfide bridge, and the amino acids believed to stabilize said bridge and to be of critical importance for the properties, that is Tyr in position 2, Asn in position 5 and Pro in position 7. In position 3 the hydrophobic Ile can be exchanged for other hydrophobic amino acids and in position 4 the hydrophilic Gln can be exchanged for other hydrophilic amino acids. In position 8 oxytocin analogues are obtained if Leu is exchanged for the hydrophobic amino acids Ile or Val, and vasopressin analogues are obtained if exchanged for the hydrophilic amino acids Arg, Hos, Cit or Daba. The nonapeptides of the invention have been compiled through methods described by S. Hellberg et al., "Peptide Quantitative Structure-Activity Relationships, a multivariate approach", J. Med. Chem. 1987, 30, 1126 and J. Jonsson et al., "Multivariate parametrization of 55 coded and non-coded amino acids", Quant. Struct.-Act. Relat., 8, 204-209 (1989). The peptides can be synthesized according to known methods (e.g. Merrifields solid phase synthesis for instance as described in Streitwieser and Heathcock, Introduction to Organic Chemistry 3^{rd} ed, p 949-950). Sequences with reversed peptide bonds can also be prepared through e.g. retro-inversomodification (see e.g. S. Müller et al, PNAS vol 94, nov 97, 12545-12550).

The use of oxytocin constitutes a preferred embodiment of the invention.

Another preferred embodiment of the invention is the use of the substances mesotocin, isotocin, vasopressin or vasotocin having the formulas:

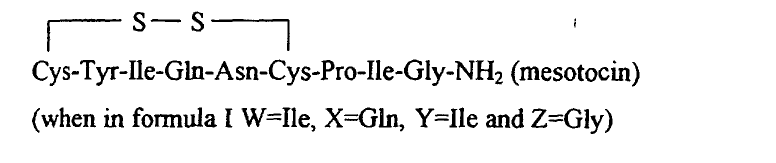

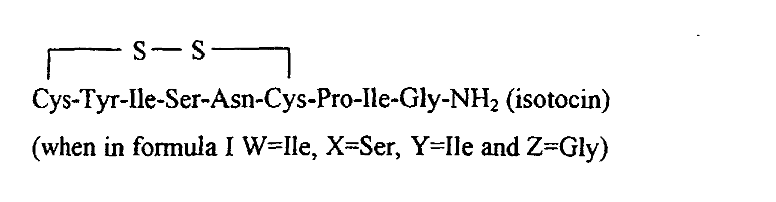

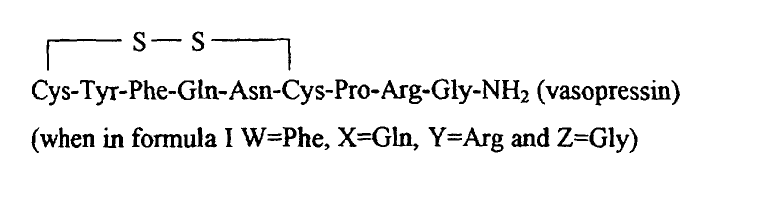

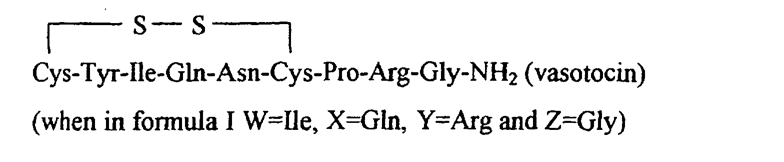

Other preferred substances for use according to the invention are: 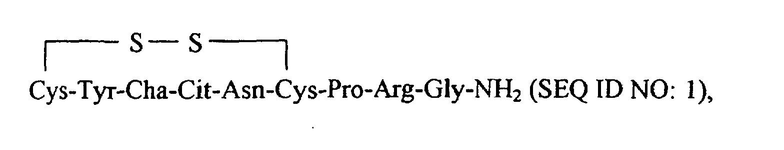 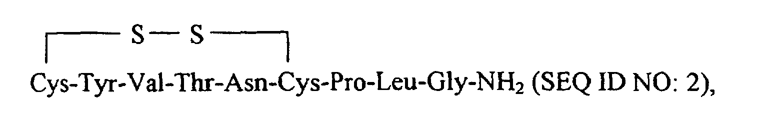 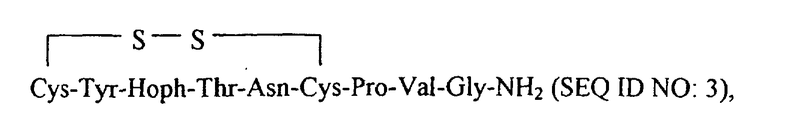 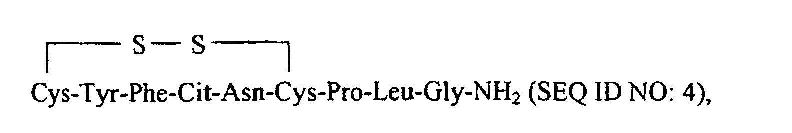 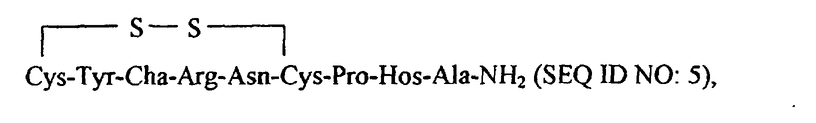 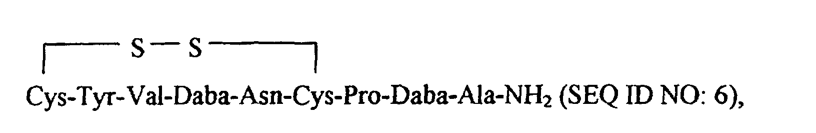 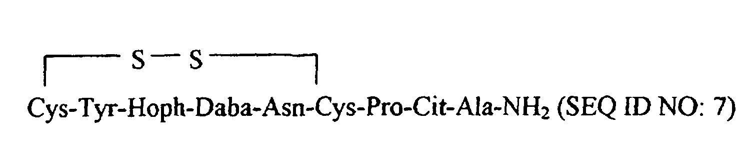 and

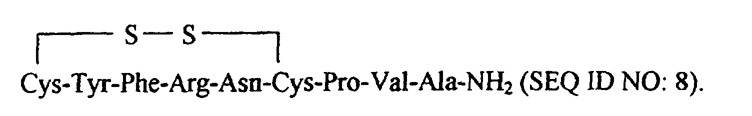

In experiments, which are described below, it has been shown that oxytocin by way of a central action increases the activity of the central α₂-receptors in rats. These receptors have an inhibitory action and counteracts the activating aspects of noradrenalin in the brain which are mainly mediated via α₁-receptors, which activate cyclic AMP. When α₂-receptor stimulation dominates over α₁-receptor stimulation, activity is exchanged by relaxation and energy is shunted towards growing and healing, i.e. is not used for stress or muscular contraction and activity. As a consequence para-sympathetic nerve tone dominates over sympathetic nervous tone and the musculature is relaxed. It can be presumed that oxytocin exerts a similar effect also in humans. During breast feeding - a situation characterized by repetitive oxytocin secretion - all the effects observed in experimental animals following repeated oxytocin administration are seen. It is not known how the effect by oxytocin on the α₂-receptors is mediated, but probably not by a classical oxytocin receptor mediated effect.

The effect of oxytocin can be extended or strengthened by administration in combination with drugs increasing the release of oxytocin and/or the number of receptors, such as estrogen, or drugs having an α₂-agonistic effect, such as clonidine.

The invention also refers to the use of oxytocin in combination with estrogen for the preparation of a pharmaceutical composition for the curative or prophylactic treatment of effective pain syndromes of non-organic origin, especially for the treatment of fibromyalgia. Oxytocin and estrogen are either administrable together or estrogen is given first and oxytocin subsequently. Estrogen increases release and synthesis of oxytocin, the synthesis of oxytocin receptors, and it also upregulates the activity of α₂-receptors.

The short term effects of oxytocin, such as short term pain relief, short term sedation and hormone release, are reversed by oxytocin antagonists and can thus be described as direct effects.

The long term effects, amounting to days-months of relief, which are obtained after repeated treatments, for 3-10 days, preferably for 5-8 days, in intervals ranging from weeks to months, are surprising not only in a pain relieving perspective but also in a general perspective and have not previously been reported for any other drug in patients. When said effect has been obtained it can be maintained by for instance once in a week treatment. This long term effect is reversed by α₂-antagonists and potentiated by α₂-agonists.

The pharmaceutical composition containing oxytocin can be formulated for topical, iontophoretical, nasal, intrapulmonary, parenteral, such as subcutaneous, intraperitoneal or intravenous, intrathecal or intracerebroventricular administration. A preferred way of administration is intranasally. A parenteral composition is for instance a solution or emulsion for subcutaneous, intramuscular or intravenous injection. A topical composition can be a lotion, cream, ointment or gel, for instance incorporated into a plaster.

Doses to be given in topical administration can be 1-1000 µg/cm², preferably 10-100 µg/cm²; in parenteral administration 0.05-1 mg/kg; and in nasal administration 1-100 IU/d, preferably 25-50 IU/d. 1 IU or internal unit corresponds to 2 µg. To children with navel colic a preferred dose might be 20-50, preferably 25-30 IU/d nasally, preferably administered 2-3 times during 1-2 hours. In i.c.v. or i.t. administration the doses are generally 1/10 to 1/1000 of the parenteral doses.

In the market there are today commercial solutions for injection and nasal sprays.

### Description of the drawings

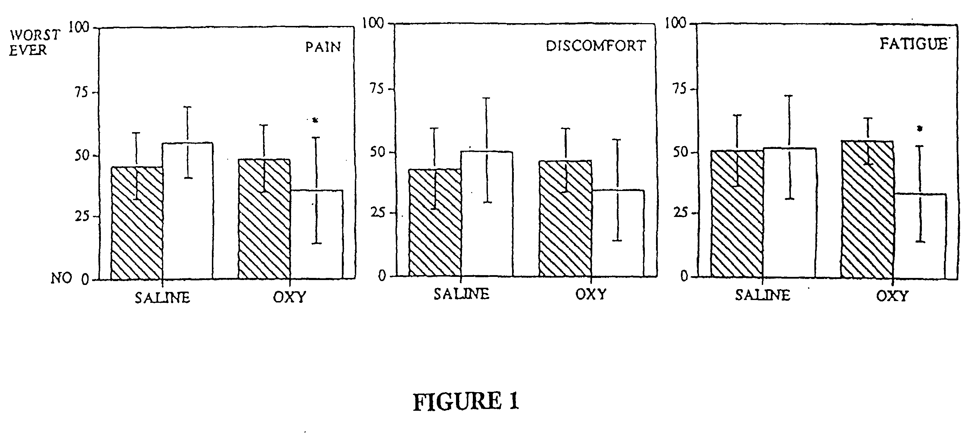

Figure 1 is a bar graph of the estimated pain, discomfort and fatigue, respectively, in fibromyalgia patients after nasal administration of oxytocin. The striped bars refer to the pain, ache-discomfort and fatigue before the oxytocin or saline administration, whereas the empty bars refer to the pain, ache-discomfort and fatigue after the oxytocin or saline administration.

### Biological tests

### Test 1. Clinical study on fibromyalgia patients

In a pilot study 12 female patients (aged 42-58 years) were treated with oxytocin (Syntocinon, Sandoz), 10 IU daily for 5 days, or saline as a control in a randomized cross-over study. All patients had previously been subjected to pharmacological treatment and/or physiotherapy with little or no alleviation and were during the trial not subjected to any other treatment modality. Before and after the trial the patients rated their pain, ache-discomfort and fatigue on visual analogue scales from 0 to 100, where 0 means no discomfort.

The results summarized in figure 1 show that 7 of 12 patients treated with oxytocin reported a significant, that is more than 50%, alleviation of their symptoms, whereas in the patients treated with saline 2 of 12 patients obtained an alleviation.

The results imply that exogenous administration of oxytocin, oxytocin agonist or a drug that releases oxytocin may alleviate the symptoms of fibromyalgia.

### Test 2. Clinical study on RAP children

3 children at the age of ten with a recurrent abdominal pain syndrome for at least 1 year were given a low dose of oxytocin by nasal spraying, that is 2 x 8 IU daily in the morning for 3-5 days. The oxytocin preparation used was Syntocinon nasal spray (Sandoz, 4 IU per spray dose).

A positive effect of the general condition and reduced pain could be observed. In one case the,pain disappeared instantly or within minutes from the administration and the patient called the drug "the miracle drops".

The results obtained are very promising and should be sufficient to justify a continued investigation in a controlled clinical study.

### Test 3. Administration of α₂-agonist to oxytocin treated rats

In order to investigate mechanisms behind the effects of oxytocin treatment on the affective component of pain clonidine - an α₂-agonist which lowers blood pressure and causes sedation - was administered (100 µg/kg s.c.) 9 days after the end of the oxytocin treatment period. At this time the significant decrease, compared to controls, had disappeared, but after the clonidine injection a significant sedation was again observed in the oxytocin-treated rats in comparison to the saline-treated controls.

These fmdings suggest that repeatedly given injections of oxytocin induce a change in the central α₂-receptors. If this change is caused by an increase in the number of receptors, a change in binding to said receptors or by some other mechanism is not yet known.

## Claims

1. Use of a substance with oxytocin activity for the preparation of a pharmaceutical composition for the treatment or prophylaxis of affective pain syndroms of non-organic origin in fibromyalgia, myofascial pain syndrome, navel colic, colitis as well as of affective components associated with pain of organic origin in mammals including man

2. Use of a substance according to claim 1, **characterized in** having the following formula I: 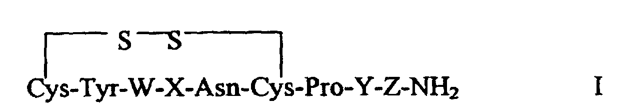 wherein
W is selected from the group consisting of Ile, Cha, Val, Hoph and Phe;
X is selected from the group consisting of Gln, Ser, Thr, Cit, Daba and Arg;
Y is selected from the group consisting of Leu, Ile, Arg, Hos, Daba, Cit and Val; where Leu, Ile and Val give oxytocin analogues and Arg, Hos, Daba and Cit give vasopressin analogues; and
Z is selected from the group consisting of Gly and Ala;
as well as sequences with reversed peptide bonds.

3. Use according to claim 1 or 2, **characterized in that** the substance is oxytocin.

4. Use according to claim 1 or 2, **characterized in that** the substance is mesotocin, isotocin, vasopressin or vasotocin.

5. Use according to claim 1 or 2, **characterized in that** the substance is selected from the group consisting of: 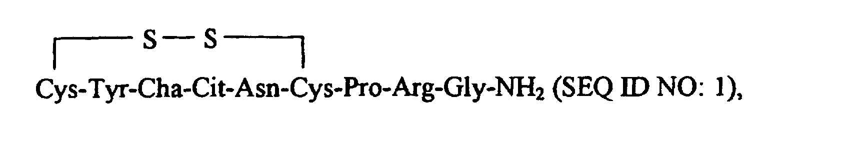 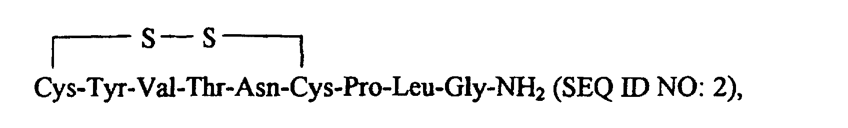 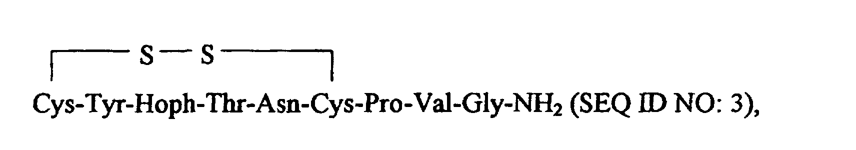 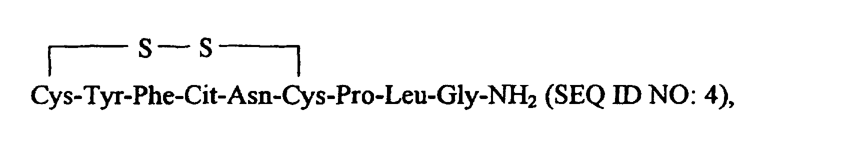 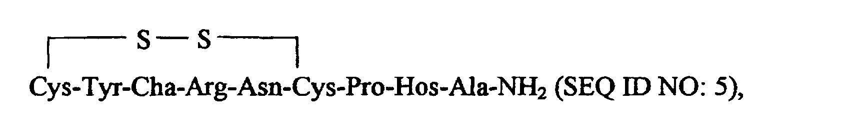 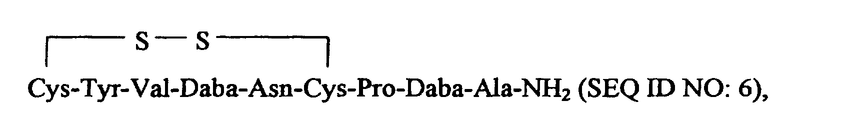 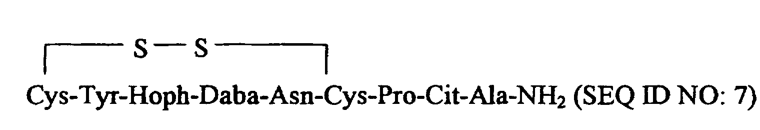 and

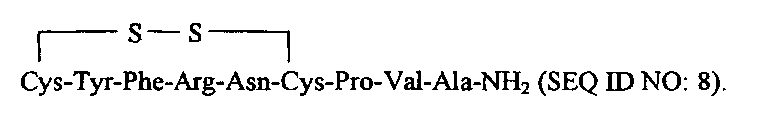

6. Use of the substance according to any of claims 1-5, for the treatment of fibromyalgia.

7. Use of the substance according to any of claims 1-5 in combination with estrogen.

8. Use of a substance according to any of claims 1-5, for the treatment of navel colic.

9. Use according to any of claims 1-8, wherein the pharmaceutical composition comprises a substance with oxytocin activity in combination with a pharmaceutically inert carrier.

10. Use according to any of claims 1-9, wherein the pharmaceutical composition is formulated for nasal, parenteral or topical administration.

11. Use according to any of claims 1-10, wherein the pharmaceutical composition is in the form of a nasal spray.

12. Use of a substance with oxytocin activity or an oxytocin agonist, especially oxytocin, for the preparation of a pharmaceutical composition for the treatment or prophylaxis of affective pain syndromes of non-organic origin, in fibromyalgia, myofascial pain syndrome, navel colic, colitis, as well as of affective components associated with pain of organic origin in mammals including man.

13. Use according to claim 12, **characterized in that** the substance with oxytocin activity is to be administered for 3-10 days, preferably for 5-8 days, in intervals ranging from weeks to months.

14. Use according to claim 12 or 13, comprising nasal administration of 5-100, preferably 25-50 IU of substance with oxytocin activity per day.

15. Use of oxytocin or a precursor, metabolic derivative, oxytocin agonist or analogue in combination with a drug having an α₂-agonistic effect for the preparation of a pharmaceutical composition for the treatment or prophylaxis of affective pain syndromes of non-organic origin, in fibromyalgia, myofascial pain syndrome, navel colic, colitis, as well as of affective components associated with pain of organic origin in mammals including man.

16. Use according to claim 15, wherein the drug having the α₂-agonistic effect is clonidine.

## Patentansprüche

1. Verwendung einer Substanz mit Oxytocin-Aktivität für die Herstellung einer pharmazeutischen Zusammensetzung für die Behandlung oder für die Prophylaxe von affektiven Schmerzsyndromen mit nichtorganischer Herkunft bei der Fibromyalgie, beim myofascialen Schmerzsyndrom, bei der Nabelkolik, bei der Dickdarmentzündung, ebenso wie von affektiven Bestandteilen, die mit Schmerz aus organischer Herkunft in Säugetieren, einschließlich Menschen, in Verbindung stehen.

2. Die Verwendung einer Substanz gemäß Anspruch 1, **dadurch gekennzeichnet, daß** sie die folgende Formel I hat: 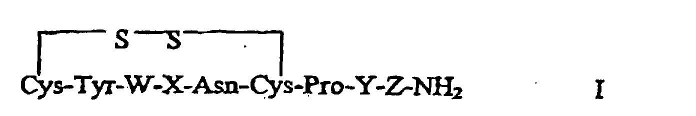 wobei
W ausgewählt ist aus der Gruppe bestehend aus Ile, Cha, Val, Hoph und Phe;
X ausgewählt ist aus der Gruppe bestehend aus Gln, Ser, Thr, Cit, Daba und Arg;
Y ausgewählt ist aus der Gruppe bestehend aus Leu, Ile, Arg, Hos, Daba, Cit und Val;
wobei Leu, Ile und Val Oxytocin-Analoga ergeben und Arg, Hos, Daba und Cit Vasopressin-Analoga ergeben; und
Z ausgewählt ist aus der Gruppe bestehend aus Gly und Ala;
ebenso wie Sequenzen mit reversen Peptidbindungen.

3. Verwendung gemäß Anspruch 1 oder 2, **dadurch gekennzeichnet, daß** die Substanz Oxytocin ist.

4. Verwendung gemäß Anspruch 1 oder 2, **dadurch gekennzeichnet, daß** die Substanz Mesotocin, Isotocin, Vasopressin oder Vasotocin ist.

5. Verwendung gemäß Anspruch 1 oder 2, **dadurch gekennzeichnet, daß** die Substanz ausgewählt ist aus der Gruppe bestehend aus: 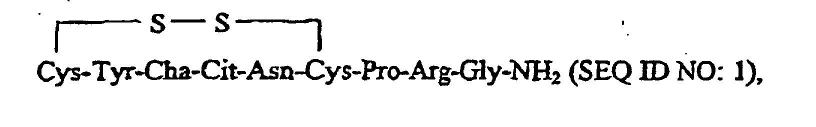 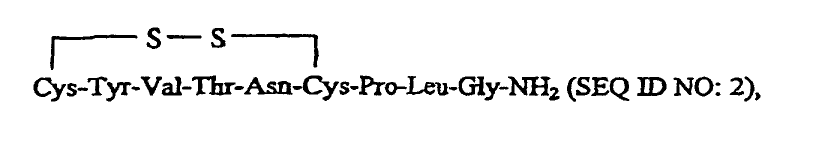 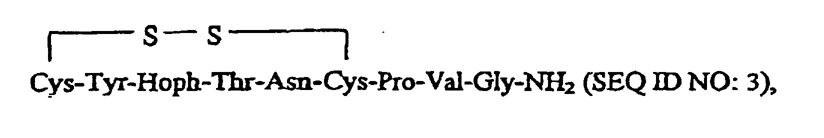 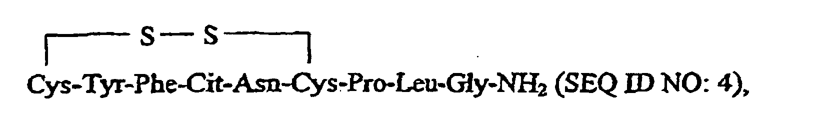 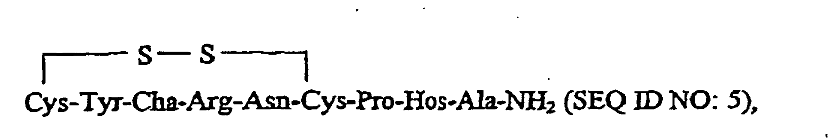 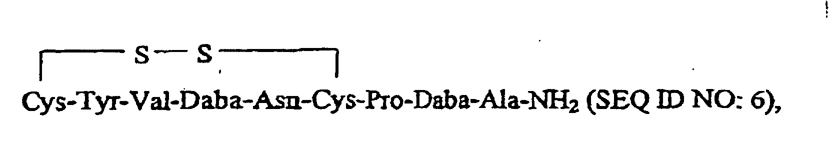 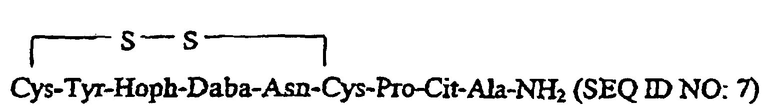 und

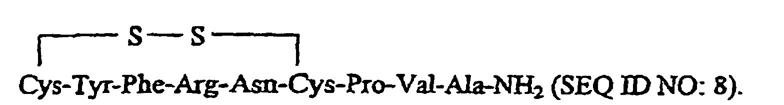

6. Verwendung der Substanz gemäß einem der Ansprüche 1-5 für die Behandlung von Fibromyalgie.

7. Verwendung einer Substanz gemäß einem der Ansprüche 1-5 in Kombination mit Östrogen.

8. Verwendung der Substanz gemäß einem der Ansprüche 1-5 für die Behandlung von Nabelkoliken.

9. Verwendung gemäß einem der Ansprüche 1-8, wobei die pharmazeutische Zusammensetzung eine Substanz mit Oxytocin-Aktivität in Kombination mit einem pharmazeutisch inerten Träger umfaßt.

10. Verwendung gemäß einem der Ansprüche 1-9, wobei die pharmazeutische Zusammensetzung für nasale, parenterale oder topische Verabreichung formuliert ist.

11. Verwendung gemäß einem der Ansprüche 1-10, wobei die pharmazeutische Zusammensetzung in der Form eines nasalen Sprays vorliegt.

12. Verwendung einer Substanz mit Oxytocin-Aktivität oder eines Oxytocin-Agonisten, insbesondere Oxytocin, für die Herstellung einer pharmazeutischen Zusammensetzung für die Behandlung oder für die Prophylaxe von affektiven Schmerzsyndromen mit nichtorganischer Herkunft bei der Fibromyalgie, beim myofascialen Schmerzsyndrom, bei der Nabelkolik, bei der Dickdarmentzündung, ebenso wie von affektiven Bestandteilen, die mit Schmerz aus organischer Herkunft in Säugetieren, einschließlich Menschen in Verbindung stehen.

13. Verwendung gemäß Anspruch 12, **dadurch gekennzeichnet, daß** die Substanz mit Oxytocin-Aktivität 3-10 Tage, vorzugsweise 5-8 Tage in Intervallen, die sich im Bereich von Wochen bis Monaten bewegen, verabreicht werden muß.

14. Verwendung gemäß Anspruch 12 oder 13, umfassend die nasale Verabreichung von 5-100, vorzugsweise 25-50 IU einer Substanz mit Oxytocin-Aktivität pro Tag.

15. Die Verwendung von Oxytocin oder einem Vorläufer, einem metabolischen Derivat, eines Oxytocin-Agonisten oder -Analogons in Kombination mit einem Medikament mit einer α2-agonistischen Wirkung für die Herstellung einer pharmazeutischen Zusammensetzung für die Behandlung oder für die Prophylaxe von affektiven Schmerzsyndromen mit nichtorganischer Herkunft bei der Fibromyalgie, beim myofascialen Schmerzsyndrom, bei der Nabelkolik, bei der Dickdarmentzündung, ebenso wie von affektiven Bestandteilen, die mit Schmerz aus organischer Herkunft in Säugetieren, einschließlich Menschen in Verbindung stehen.

16. Verwendung gemäß Anspruch 15, wobei das Medikament, das die α2-agonistische Wirkung aufweist, Clonidin ist.

## Revendications

1. Utilisation d'une substance à activité ocitocique pour la préparation d'une composition pharmaceutique destinée au traitement ou à la prophylaxie de syndromes douloureux affectifs d'origine non organique dans la fibromyalgie, le syndrome douloureux myofascial, la colique ombilicale, la colite, ainsi que des composants affectifs associés à la douleur d'origine organique chez les mammifères comprenant l'homme.

2. Utilisation d'une substance selon la revendication 1, **caractérisée en ce qu'**elle a la formule 1 suivante : 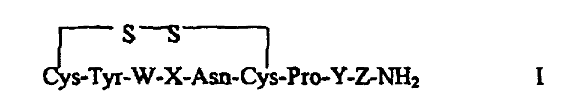 dans laquelle
W est choisi dans le groupe constitué par Ile, Cha, Val, Hoph et Phe ;
X est choisi dans le groupe constitué par Gln, Ser, Thr, Cit, Daba et Arg ;
Y est choisi dans le groupe constitué par Leu, Ile, Arg, Hos, Daba, Cit et Val ;
où Leu, Ile et Val donnent des analogues de l'ocitocine et Arg, Hos, Daba et Cit donnent des analogues de la vasopressine ; et
Z est choisi dans le groupe constitué par Gly et Ala ;
ainsi que des séquences avec des liaisons peptidiques inversées.

3. Utilisation selon la revendication 1 ou 2, **caractérisée en ce que** la substance est l'ocitocine.

4. Utilisation selon la revendication 1 ou 2, **caractérisée en ce que** la substance est la mésotocine, l'isotocine, la vasopressine ou la vasotocine.

5. Utilisation selon la revendication 1 ou 2, **caractérisée en ce que** la substance est choisie dans le groupe constitué par : 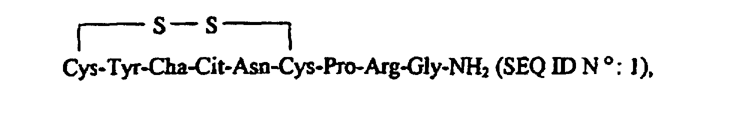 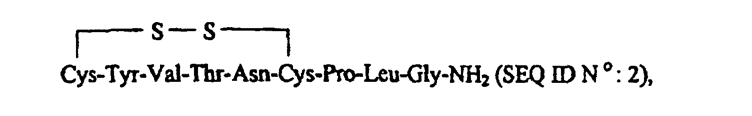 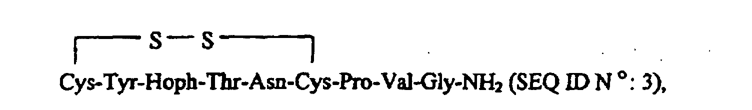 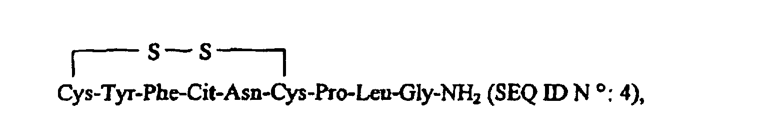 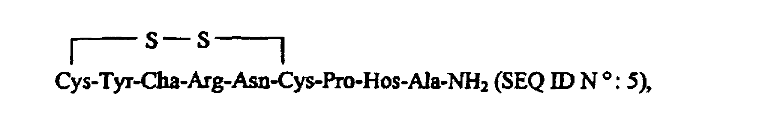 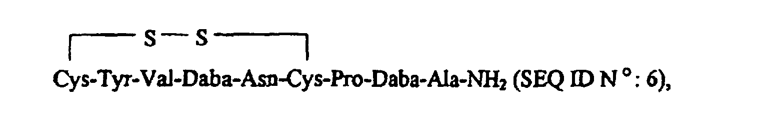 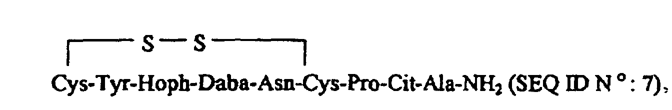 et

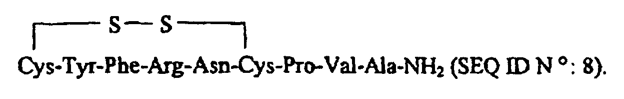

6. Utilisation de la substance selon l'une quelconque des revendications 1 à 5, pour le traitement de la fibromyalgie.

7. Utilisation de la substance selon l'une quelconque des revendications 1 à 5, en combinaison avec un oestrogène.

8. Utilisation d'une substance selon l'une quelconque des revendications 1 à 5, pour le traitement de la colique ombilicale.

9. Utilisation selon l'une quelconque des revendications 1 à 8, dans laquelle la composition pharmaceutique comprend une substance à activité ocitocique en combinaison avec un véhicule inerte sur le plan pharmaceutique.

10. Utilisation selon l'une quelconque des revendications 1 à 9, dans laquelle la composition pharmaceutique est formulée pour une administration par voie nasale, parentérale ou topique.

11. Utilisation selon l'une quelconque des revendications 1 à 10, dans laquelle la composition pharmaceutique se trouve sous la forme d'un spray nasal.

12. Utilisation d'une substance à activité ocitocique ou un agoniste de l'ocitocine, spécialement l'ocitocine, pour la préparation d'une composition pharmaceutique destinée au traitement ou à la prophylaxie des syndromes douloureux affectifs d'origine non organique, dans la fibromyalgie, le syndrome douloureux myofascial, la colique ombilicale, la colite, ainsi que des composants affectifs associés à la douleur d'origine organique chez les mammifères comprenant l'homme.

13. Utilisation selon la revendication 12, **caractérisée en ce que** la substance à activité ocitocique doit être administrée pendant 3 à 10 jours, de préférence pendant 5 à 8 jours, à des intervalles se trouvant dans la plage allant de semaines à des mois.

14. Utilisation selon la revendication 12 ou 13, comprenant une administration par voie nasale de 5 à 100, de préférence de 25 à 50 UI de substance à activité ocitocique par jour.

15. Utilisation d'ocitocine ou d'un précurseur, dérivé métabolique, agoniste ou analogue de l'ocitocine en combinaison avec un médicament ayant un effet α₂-agoniste pour la préparation d'une composition pharmaceutique destinée au traitement ou à la prophylaxie des syndromes douloureux affectifs d'origine non organique, dans la fibromyalgie, le syndrome douloureux myofascial, la colique ombilicale, la colite, ainsi que des composants affectifs associés à la douleur d'origine organique chez les mammifères comprenant l'homme.

16. Utilisation selon la revendication 15, dans laquelle le médicament ayant l'effet α₂-agoniste est la clonidine.
